(19) Europäisches Patentamt / European Patent Office / Office européen des brevets

(11) **EP 4 181 991 B1**

(12) **EUROPÄISCHE PATENTSCHRIFT**

(45) Veröffentlichungstag und Bekanntmachung des Hinweises auf die Patenterteilung:
**25.02.2026 Patentblatt 2026/09**

(21) Anmeldenummer: **21730869.1**

(22) Anmeldetag: **02.06.2021**

(51) Internationale Patentklassifikation (IPC):
**A61M 16/00** *(2006.01)*

(52) Gemeinsame Patentklassifikation (CPC):
**A61M 16/0045; A61M 16/024;** A61M 2016/0027;
A61M 2205/3344; A61M 2230/46

(86) Internationale Anmeldenummer:
**PCT/EP2021/064749**

(87) Internationale Veröffentlichungsnummer:
**WO 2022/012812 (20.01.2022 Gazette 2022/03)**

(54) **BEATMUNGSVORRICHTUNG ZUR BESTIMMUNG ZUMINDEST EINES ALVEOLÄREN DRUCKES BZW. EINES VERLAUFS EINES ALVEOLÄREN DRUCKES IN EINEM ATEMWEG EINES PATIENTEN**

VENTILATOR FOR DETERMINING AT LEAST ONE ALVEOLAR PRESSURE AND/OR A PROFILE OF AN ALVEOLAR PRESSURE IN A RESPIRATORY TRACT OF A PATIENT

VENTILATEUR POUR DÉTERMINER AU MOINS UNE PRESSION ALVÉOLAIRE ET/OU UN PROFIL D'UNE PRESSION ALVÉOLAIRE DANS LES VOIES RESPIRATOIRES D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.07.2020 DE 102020118529**

(43) Veröffentlichungstag der Anmeldung:
**24.05.2023 Patentblatt 2023/21**

(73) Patentinhaber: **Ventinova Technologies B.V.**
**5652 BJ Eindhoven (NL)**

(72) Erfinder:
• **ENK, Dietmar**
**48653 Coesfeld (DE)**
• **BARNES, Thomas Heinrich**
**Warlingham Surrey CR6 9LB (GB)**

(74) Vertreter: **karo IP**
**Patentanwälte PartG mbB**
**Steinstraße 16-18**
**40212 Düsseldorf (DE)**

(56) Entgegenhaltungen:
• **DAVID W. KACZKA ET AL: "Oscillation Mechanics of the Respiratory System: Applications to Lung Disease", CRITICAL REVIEWS(TM) IN BIOMEDICAL ENGINEERING, vol. 39, no. 4, 1 January 2011 (2011-01-01), pages 337 - 359, XP055157519, ISSN: 0278-940X, DOI: 10.1615/CritRevBiomedEng.v39.i4.60**
• **STANKIEWICZ BARBARA ET AL: "A new infant hybrid respiratory simulator: preliminary evaluation based on clinical data", MEDICAL AND BIOLOGICAL ENGINEERING AND COMPUTING, SPRINGER, HEILDELBERG, DE, vol. 55, no. 11, 25 March 2017 (2017-03-25), pages 1937 - 1948, XP036341840, ISSN: 0140-0118, [retrieved on 20170325], DOI: 10.1007/S11517-017-1635-9**
• **MARCHAL ET AL: "Forced oscillations, interrupter technique and body plethysmography in the preschool child", PAEDIATRIC RESPIRATORY REVIEWS, W.B. SAUNDERS, AMSTERDAM, NL, vol. 6, no. 4, 1 December 2005 (2005-12-01), pages 278 - 284, XP005166861, ISSN: 1526-0542, DOI: 10.1016/J.PRRV.2005.09.007**

**Beschreibung**

Die Erfindung betrifft eine Beatmungsvorrichtung zur Ermittlung bzw. Bestimmung von Kennwerten bzw. zumindest eines (globalen) alveolären Druckes bzw. eines Verlaufs eines alveolären Druckes (in den Lungenbläschen) in einem Atemweg eines Patienten, ggf. zusätzlich zur Ermittlung bzw. Bestimmung einer atemwegsrelatierten Resistance und / oder einer gewebsrelatierten Resistance.

Der inspiratorische Spitzendruck oder "peak inspiratory pressure" (PIP) bezeichnet den höchsten positiven Druck [in mbar, also Millibar], der während der Einatmung (Inspiration) künstlich im Atemweg erzeugt wird.

Der endinspiratorische (Plateau-)Druck ist der Druck, der am Ende der Inspiration im Atemweg gemessen wird.

Der endexspiratorische (Plateau-)Druck, der nach Abschluss der Ausatmung (Exspiration) im Atemweg gehalten wird, ist vorzugsweise positiv und wird daher auch als positiver endexspiratorischer Druck oder "positive end-expiratory pressure" (PEEP) bezeichnet. Im Folgenden wird immer auf den PEEP verwiesen.

Die Compliance [in ml / mbar, also Milliliter / Millibar] ist ein Maß für die Dehnbarkeit oder auch Druckelastizität des Lunge-Brustkorb-Systems eines Patienten. Unter Beatmungsbedingungen wird für die Berechnung der sogenannten statischen Compliance das Tidalvolumen ($V_T$) [in ml], das das während einer Inspiration zugeführte Luftvolumen bezeichnet, und die Differenz von endinspiratorischem (Plateau-)Druck (z. B. $P_{I1}$) und endexspiratorischem (Plateau-)Druck (z. B. $P_{E1}$) herangezogen.

Hingegen wird die sogenannte dynamische Compliance auf der Basis des $V_T$ und der Differenz von PIP (z. B. $P_{I2}$) und PEEP (z. B. $P_{E2}$) [in mbar] berechnet. Die Druckdifferenz ist daher bei der dynamischen Compliance regelmäßig größer oder zumindest gleich groß wie die Druckdifferenz bei der statischen Compliance. Da die Compliance im Allgemeinen ein sich - mit sich änderndem Druck (P) und Volumen (V) - änderndes Verhältnis von Druck und Volumen aufweist, stellt sie sich in einem Druck-Volumen-Diagramm als Kurve dar.

Die Compliance gibt also an, wie viel Fluid (z. B. Atemgas, also ein Luftvolumen), also ein delta V, in den mindestens einen Atemweg eingeführt oder aus dem Atemweg entfernt wird, so dass sich ein Druck in dem Atemweg um eine Druckdifferenz delta P verändert. Während mindestens eines Beatmungsvorgangs (umfassend einen Inspirationsvorgang, also das Zuführen von Fluid in den Atemweg, sowie einen Exspirationsvorgang, also das Abführen von Fluid aus dem Atemweg) kann ein Verlauf der Compliance-Kurve ermittelt oder zusätzlich abgeschätzt werden (z. B. aufgrund von Erfahrungswerten). Dabei kann insbesondere der Teilbereich der Compliance-Kurve ermittelt werden, bei dem ein bestimmtes Volumen (ggf. $V_T$) in einem möglichst kleinen Druckintervall zugeführt werden kann.

Ein Anwender oder vorzugsweise eine (automatisch arbeitende) Steuereinrichtung einer Beatmungsvorrichtung kann so, unter Berücksichtigung eines ermittelten oder zusätzlich abgeschätzten Verlaufs zumindest eines Teilbereichs einer Compliance-Kurve in einem Druck-Volumen-Diagramm, eine Lage eines Druckintervalls mit den Drücken $P_I$ und $P_E$ bestimmen und diese Drücke dann an der Beatmungsvorrichtung einstellen (z. B. PIP als $P_I$ und PEEP als $P_E$), so dass zumindest ein Beatmungsvorgang, also eine Inspiration und / oder eine Exspiration, zwischen diesen Drücken $P_I$ und $P_E$ erfolgt und ein Betrag der Compliance dieses Beatmungsvorgangs möglichst groß ist.

Die fortlaufende Beatmung sollte dabei so eingestellt werden, dass ein für eine Normoventilation (also die adäquate Elimination bzw. Abatmung von Kohlendioxid) erforderliches Minutenvolumen (also $V_T$ • Beatmungsfrequenz [/min, also Beatmungsvorgänge pro Minute]) möglichst klein ist und bei möglichst maximaler Compliance zu- und abgeführt werden kann.

Im Gegensatz zur statischen Compliance fließen in die dynamische Compliance zwangsläufig auch die während der In- bzw. Exspiration zu überwindenden Widerstände (im weitesten Sinne) einschließlich von Effekten der sogenannten Beatmungshistorie (also wie die Lunge beatmet wurde) mit ein. Letzteres ergibt sich aus dem Umstand, dass es sich bei der Lunge um ein viskoelastisches Organ handelt, dessen mechanische Eigenschaften davon abhängen, wie es bewegt wird bzw. wurde.

Die Resistance (gemessen in mbar / l / s; also Millibar / Liter / Sekunde bzw. mbar • s / l, also Millibar • Sekunde / Liter) beschreibt die während der In- bzw. Exspiration zu überwindenden Widerstände und gibt den Druck an, der für den Gasfluss (Fluidstrom) und damit die Volumenänderung (in der Lunge) pro Zeit notwendig ist.

Bei einem beatmeten Patienten wird die Resistance typischerweise während der Inspiration durch Messung der Druckdifferenz zwischen inspiratorischem Spitzendruck (PIP) und endinspiratorischem (Plateau-)Druck in Relation zum mittleren inspiratorischen Fluss (inspiratorischen Fluidstrom) abgeschätzt. Voraussetzung für die Messung ist (bisher) ein Stopp des inspiratorischen Flusses.

So ergibt sich z. B. aus einer Druckdifferenz zwischen inspiratorischem Spitzendruck ($P_{I2}$) und endinspiratorischem (Plateau-)Druck ($P_{E2}$) von 2 mbar bei einem mittleren inspiratorischen Fluss von 18 l / min [Liter / Minute] eine (inspiratorische) Resistance von 6,67 mbar / l / s; also 2 mbar / 18 l / min bzw. 2 mbar • 60 s / 18 l.

In konventionellen Beatmungsgeräten wird die Resistance zumeist mit diesem Verfahren bestimmt. Daneben gibt es verschiedene andere, auf intermittierenden oder superponierten Messungen beruhende Verfahren zur Bestimmung der Resistance.

Um die Eigenschaften einer beatmeten Lunge vollumfänglich erfassen und beschreiben zu können, ist neben einer präzisen Messung der dynamischen Compliance auch eine genaue Bestimmung der in- und exspiratorischen Resistance wünschenswert. Dies ist eine Voraussetzung, um insbesondere kritisch (lungen-) kranke Patienten individualisiert und möglichst schonend im Bereich optimaler Compliance beatmen zu können, also zwischen einem sogenannten unteren Inflektionspunkt, an dem die Compliance inspiratorisch im Sinne optimaler Rekrutierung des Lungengewebes maximal zunimmt und einem sogenannten oberen Inflektionspunkt, an dem die Compliance inspiratorisch aufgrund zunehmender Überdehnung im Lungengewebe maximal abnimmt.

Die Resistance ergibt sich aber nicht nur aus dem gasflussabhängigen Widerstand der Luftwege (atemwegsrelatiert; z. B. Querschnitt der Atemwege, Turbulenzen), sondern auch aus Widerständen im Gewebe (gewebsrelatiert; z. B. durch Scherung, Friktion, Viskoelastizität, ggf. durch Massenträgheit).

Massenträgheitseffekte spielen insbesondere zu Beginn der Inspiration und Exspiration eine Rolle, wenn durch die Zu- bzw. Abnahme des Lungenvolumens Gewebe (sowohl der Lunge selbst als auch umgebendes/ angrenzendes Gewebe) beschleunigt und abgebremst werden muss. Zu Scherung kann es innerhalb (insbesondere funktionell inhomogenen) Lungengewebes kommen (sogenannter "shear stress"), zu Friktion hingegen an Grenzflächen wie z. B. der Verschiebeschicht von Brust- und Lungenfell (Pleura parietalis und Pleura visceralis), die inspiratorisch zudem von der (Flächen-)Größe zu- und exspiratorisch von der (Flächen-)Größe wieder abnimmt. Viskoelastische Effekte ergeben sich u.a. aus einem in- und exspiratorisch unterschiedlichen Blutvolumen in der Lungenstrombahn, wodurch die Lunge unterschiedlich resistiv wird.

Innerhalb der Lunge gibt es nicht nur unterschiedlich kompliante (dehnbare) Lungenkompartimente, sondern auch bezüglich der Resistance finden sich Lungenkompartimente, die eine niedrigere oder höhere atemwegs- oder gewebsrelatierte Resistance aufweisen. Daraus ergibt sich notwendigerweise, dass man von außen immer nur ein globales Bild der Compliance und der Resistance bekommt.

Eine Differenzierung der Resistance in einen atemwegs- und gewebsrelatierten Teil ist aber klinisch interessant: Auf der Basis des atemwegsrelatierten Teils kann grundsätzlich der (globale) alveoläre Druckverlauf (in den Lungenbläschen) berechnet werden. Zudem ist eine erhöhte atemwegsrelatierte Resistance (im Gegensatz zum gewebsrelatierten Teil) einer medikamentösen Therapie zugänglich. Hingegen weist die gewebsrelatierte Resistance auf Veränderungen im (Lungen-)Gewebe hin. Damit bietet sich die gewebsrelatierte Resistance als diagnostischer, therapeutischer oder sogar prognostischer Parameter an.

In der Literatur wird der Anteil der gewebsrelatierten Resistance an der auf (inspiratorischem) Spitzendruck gemessenen (Gesamt-)Resistance auf etwa 25 % geschätzt, d. h. etwa 75 % der (Gesamt-)Resistance entfällt auf den atemwegsrelatierten Teil. Diesen Angaben liegen zumeist invasive Messverfahren (z. B. Ösophagusdruckmessungen) zugrunde.

Die Veröffentlichung von David W. Kaczka et al "Oscillation Mechanics of the Respiratory System: Applications to Lung Disease" (01-01-2011) ist auf das Thema Atemwege und Beatmung gerichtet.

Die Veröffentlichung Stankiewicz Barbara et al. *"A new infant hybrid respiratory simulator: preliminary evaluation based on clinical data"* (25-03-2017) ist auf das Thema Beatmungsvorrichtung für Kleinkinder gerichtet.

Aufgabe der vorliegenden Erfindung ist es, die mit Bezug auf den Stand der Technik angeführten Probleme (zumindest teilweise) zu lösen. Insbesondere soll eine Beatmungsvorrichtung zur Bestimmung von Kennwerten bzw. zumindest eines (globalen) alveolären Druckes bzw. eines Verlaufs eines alveolären Druckes (in den Lungenbläschen) in einem Atemweg eines Patienten, ggf. zusätzlich zur Ermittlung bzw. Bestimmung einer atemwegsrelatierten Resistance und / oder einer gewebsrelatierten Resistance mit einer Beatmungsvorrichtung vorgeschlagen werden. Insbesondere soll eine Beatmungsvorrichtung vorgeschlagen werden, durch die Kennwerte festlegbar und bestimmbar sind.

Zur Lösung dieser Aufgaben trägt eine Beatmungsvorrichtung mit den Merkmalen gemäß Patentanspruch 1 bei. Vorteilhafte Weiterbildungen sind Gegenstand der abhängigen Patentansprüche. Die in den Patentansprüchen einzeln aufgeführten Merkmale sind in technologisch sinnvoller Weise miteinander kombinierbar und können durch erläuternde Sachverhalte aus der Beschreibung und / oder Details aus den Figuren ergänzt werden, wobei weitere Ausführungsvarianten der Erfindung aufgezeigt werden.

Es wird eine Beatmungsvorrichtung vorgeschlagen, zumindest umfassend eine Gaszuführeinrichtung und eine Gasabführeinrichtung, zum Zuführen eines (inspiratorischen) Fluidstroms hin zu einem Atemweg eines Patienten und zum Abführen eines (exspiratorischen) Fluidstroms aus dem Atemweg (des Patienten) zurück in die Beatmungsvorrichtung oder an eine Umgebung, einen Drucksensor zur Erfassung eines Druckes in dem Atemweg sowie eine Steuereinrichtung zum Betreiben der Beatmungsvorrichtung.

Insbesondere ist ein Fluidstrom zumindest während eines Inspirationsvorgangs und eines Exspirationsvorgangs auf einen vorbestimmbaren Wert einstellbar. Die Steuereinrichtung ist zum Betreiben der Beatmungsvorrichtung und zur Durchführung eines Verfahrens, insbesondere eines Mess- und Berechnungsverfahrens, eingerichtet, das zumindest die folgenden Schritte umfasst

a) Festlegen eines Druckintervalls, in dem der Patient für ein festgelegtes Zeitintervall zu beatmen ist;

b) wiederholtes abwechselndes Durchführen von jeweils einem Inspirationsvorgang mit einem ersten Fluidstrom $Q_1$ mittels der Gaszuführeinrichtung und von jeweils einem Exspirationsvorgangs mit einem zweiten Fluidstrom $Q_2$ mittels der Gasabführeinrichtung innerhalb des Druckintervalls,

c) Erfassen des sich während Schritt b) verändernden Druckes und der Fluidströme;

d) Durchführen einer Fouriertransformation für die erfassten Werte des Druckes und Bilden eines ersten Frequenzspektrums für den Druck sowie Durchführen einer Fouriertransformation für die erfassten Werte der Fluidströme und Bilden eines zweiten Frequenzspektrums für die Fluidströme;

e) Berechnen einer Impedanz $Z_{aw}$ des Atemwegs durch Dividieren des ersten Frequenzspektrums durch das zweite Frequenzspektrum, wobei die Impedanz einen realen Anteil $Real(Z_{aw})$ und einen imaginären Anteil $Im(Z_{aw})$ aufweist;

f) Nachbilden zumindest des realen Anteils durch ein mathematisches erstes Modell und Ermitteln eines alveolären Druckes bzw. eines Verlaufs eines alveolären Druckes.

Insbesondere erfolgt bei der hier vorgeschlagenen Beatmungsvorrichtung eine Beatmung des Patienten ausschließlich über die Beatmungsvorrichtung. Insbesondere wird also der Atemweg des Patienten ausschließlich über die Beatmungsvorrichtung mit dem Fluidstrom beaufschlagt (während der In- und Exspiration). Es liegt also insbesondere kein Fluidstrom vor, der nicht von der Beatmungsvorrichtung initiiert oder generiert wird. Insbesondere umfasst die Beatmungsvorrichtung dafür ein Lumen für die Inspiration und ein Lumen für die Exspiration. Insbesondere ist ein gemeinsames Lumen (z. B. ein Beatmungskatheter) vorgesehen, so dass der Fluidstrom nur durch ein Lumen dem Atemweg zugeführt oder aus dem Atemweg abgeführt wird.

Insbesondere ist der Drucksensor endotracheal (also in der Luftröhre) angeordnet. Damit kann der Druck innerhalb des Atemwegs des Patienten ermittelt werden.

Insbesondere ist der Drucksensor am distalen Ende eines Beatmungskatheters angeordnet, der sich als Bestandteil der Beatmungsvorrichtung in dem Atemweg des Patienten befindet.

Der Drucksensor kann ggf. auch nicht endotracheal, sondern beabstandet vom Patienten angeordnet sein. Der tracheale Druck sollte dann rechnerisch bestimmbar sein. Allerdings können sich bei einer derartigen Anordnung des Drucksensors Ungenauigkeiten ergeben, die die hier beschriebenen Messungen beeinträchtigen können.

Der Beatmungskatheter weist insbesondere ein Totraumvolumen (also das Volumen, dass während eines Inspirations- oder Exspirationsvorgangs in dem Beatmungskatheter verbleibt) von höchstens 100 ml auf, insbesondere von höchstens 50 ml.

Insbesondere werden die in Schritt c) erfassten Werte des Druckes und der Fluidströme ausschließlich anhand des Beatmungsvorgangs (umfassend einen Inspirationsvorgang und einen Exspirationsvorgang) erfasst, d. h. es erfolgt insbesondere keine zusätzliche Anregung bzw. Veränderung des Druckes oder der Fluidströme durch eine nicht ausschließlich der Beatmung dienende Vorrichtung.

Es ist z. B. bekannt, dass der Atemweg bzw. eine Beatmungsvorrichtung mit einem Signal angeregt werden können, so dass dadurch hervorgerufene Schwankungen des Druckes innerhalb des Atemwegs erfasst werden können. Dabei erfolgt diese Anregung jedoch außerhalb des eigentlichen Beatmungsvorgangs, also nicht während des Vorliegens eines Fluidstroms. Weiterhin ist zur Erzeugung des Signals eine eigene Vorrichtung bereitzustellen bzw. ein zusätzlicher apparativer Aufwand erforderlich.

Insbesondere wird vorliegend berücksichtigt, dass z. B. eine atemwegsrelatierte Resistance und eine gewebsrelatierte Resistance sich im Hinblick auf eine Beatmungsfrequenz unterschiedlich verändern. Dabei kann insbesondere angenommen werden, das z. B. die atemwegsrelatierte Resistance im Wesentlichen unabhängig von der Beatmungsfrequenz ist, sich also bei sich ändernder Beatmungsfrequenz nicht verändert.

Insbesondere werden vorliegend Frequenzspektren für die erfassten Werte des Druckes und der Fluidströme gebildet, d. h. die in Schritt c) im Zeitbereich erfassten Werte werden im Rahmen des Schrittes d) durch eine Fouriertransformation in den Frequenzbereich transformiert.

Die Qualität dieser Transformation kann insbesondere durch die Art der Beatmung beeinflusst werden. Bestimmte Beatmungsverfahren und dafür eingerichtete Beatmungsvorrichtungen sind daher in besonders vorteilhafter Weise zur Durchführung dieses Verfahrens geeignet.

Im Rahmen des Schrittes e) kann eine Impedanz aus den Frequenzspektren berechnet werden. Diese umfasst insbesondere einen realen und einen imaginären Anteil, ist also eine komplexe Größe. Diese berechnete Impedanz bzw. deren Verlauf über die Frequenz, insbesondere deren realer Anteil wird

im Rahmen des Schrittes f) durch ein mathematisches erstes Modell nachgebildet.

Da der Verlauf durch das mathematische erste Modell nachgebildet ist, werden mit einer vorbestimmbaren Genauigkeit aus dem ersten Modell bestimmte Parameter bzw. Kennwerte ausgelesen. Insbesondere wird aus dem realen Anteil der alveoläre Druck bzw. dessen Verlauf bestimmt.

Das erste Modell umfasst die Gleichung

$$Real(Z_{aw}) = R_{aw} + \frac{G}{\omega^\alpha}$$ , mit $R_{aw}$: atemwegsrelatierte Resistance; $\frac{G}{\omega^\alpha}$ : gewebsrelatierte Resis-

tance; mit G als einer Konstanten, $\omega$ der Kreisfrequenz (also 2 x $\pi$ x Frequenz der Beatmung) und $\alpha$ als einer Konstanten; wobei der reale Anteil die Resistance, also die während der In- bzw. Exspiration zu überwindenden Widerstände, beschreibt.

Dabei ist die Kreisfrequenz $\omega$ insbesondere die Beatmungsfrequenz (d. h. die Anzahl Beatmungsvorgänge pro Zeiteinheit während Schritt b)) multipliziert mit dem Faktor 2 x $\pi$.

Der Verlauf des gemäß Schritt e) berechneten realen Anteils der Impedanz wird durch die Kennwerte bzw. Parameter des ersten Modells mathematisch nachgebildet. Dabei können bekannte Näherungsverfahren eingesetzt werden.

Der alveoläre Druck $P_{alv}$ bzw. dessen Verlauf wird aus der Gleichung $P_{alv} = P_{trach} - Q_i \times R_{aw}$ ermittelt, mit $Q_i$: dem jeweils während Schritt b) vorliegenden Fluidstrom.

Der gemessene oder bestimmte Druck $P_{trach}$ ist der über die Zeit sich verändernde Druck in dem Atemweg. Der Verlauf des Druckes $P_{alv}$ über die Zeit ergibt sich also in Abhängigkeit von dem sich über die Zeit ändernden Druck $P_{trach}$.

Es ist beobachtet worden, dass sich die (Gesamt-)Resistance, also die Summe aus atemwegsrelatierter Resistance und gewebsrelatierter Resistance, bei Zunahme des $V_T$ aber gleichem Gaszufluss erhöht, mutmaßlich durch Zunahme der gewebsrelatierten Resistance. Dies erscheint aus folgendem Grund plausibel: Der geringe Druckfall von den kleinen Bronchien bzw. Bronchiolen (mit glatter Muskulatur), die maßgeblich die atemwegsrelatierte Resistance bestimmen, zu den Alveolen (Lungenbläschen) macht eine von den Beatmungsdrücken weitgehend unabhängige atemwegsrelatierte Resistance annehmbar, da höhere Drücke in den kleinen Bronchien bzw. Bronchiolen (mit glatter Muskulatur) notwendigerweise auch mit höheren Drücken in den abhängigen, die kleinen Bronchien bzw. Bronchiolen (mit glatter Muskulatur) umgebenden Alveolen einhergehen.

Insbesondere scheint die atemwegsrelatierte Resistance während des Inspirationsvorgangs aber eher leicht abzunehmen. Dies kann man durch eine (wenngleich geringe) Querschnittsvergrößerung der Bronchien bei steigendem Druck erklären, was den widerstandserhöhenden Effekt einer während der Inspiration zunehmenden Länge der kleinen Bronchien bzw. Bronchiolen (mit glatter Muskulatur) mehr als kompensiert. Diesbezüglich sei auf das Hagen-Poiseuille-Gesetz verwiesen, das beschreibt, dass sich Änderungen des Radius in der vierten Potenz, Änderungen der Länge aber lediglich proportional auf den Gasfluss (Fluidstrom) auswirken.

Auch wenn notwendigerweise die Höhe des Druckfalls von den kleinen Bronchien bzw. Bronchiolen (mit glatter Muskulatur) zu den Alveolen gasflussabhängig ist und es somit bei höheren Gasflüssen zu einer relativ stärkeren Dehnung und damit Querschnittsvergrößerung der kleinen Bronchien bzw. Bronchiolen (mit glatter Muskulatur) kommen kann, so führen höhere Atemwegsdrücke also allenfalls zu einer Abnahme, nicht aber Zunahme der atemwegsrelatierten Resistance.

Es ist daher plausibel anzunehmen, dass die Zunahme der inspiratorischen (Gesamt-)Resistance bei höheren Tidalvolumina aber gleichem Gaszufluss (Fluidstrom während Inspiration) maßgeblich auf einer Zunahme der gewebsrelatierten Resistance beruht. Umgekehrt darf man erwarten, dass bei exspiratorischem Abfluss des Atemgases (Fluidstrom während Exspiration) und dadurch abnehmendem Lungenvolumen der Anteil der gewebsrelatierten Resistance an der (Gesamt-)Resistance wieder abnimmt und bei vollständiger Exspiration schließlich (nahezu) verschwindet bzw. bei PEEP ein Minimum erreicht.

Insbesondere ist in Schritt f) durch ein mathematisches zweites Modell auch der imaginäre Anteil nachbildbar, wobei das zweite Modell die Gleichung

$$Z_{aw} =$$

$$R_{aw} + k \times j \times \omega \times I_{aw} + \frac{G - j \times H}{\omega^\alpha}$$

umfasst; wobei

$$Im(Z_{aw}) = j \times (k \times \omega \times I_{aw} + \frac{-H}{\omega^\alpha};$$

mit

k: einer Konstanten;
$I_{aw}$: Trägheit des Atemwegs;
$\frac{-H}{\omega^\alpha}$ : Elastizität des Atemwegs mit H als einer Konstanten;
wobei der imaginäre Anteil die Atemwegsreactance $X_a$ beschreibt, wobei eine Compliance des Atemwegs durch $C = -\frac{1}{\omega \times X_a}$ beschrieben ist.

Der imaginäre Anteil ist für die Bestimmung des alveolären Druckes bzw. dessen Verlaufs nicht zu ermitteln. Allerdings können aus dem imaginären Anteil weitere ggf. als relevant erachtete Parameter abgeleitet werden, z. B. die Atemwegsreactance und die Compliance des Atemwegs.

Insbesondere ist der Drucksensor endotracheal angeordnet.

Insbesondere werden zumindest die Schritte a) bis c) in verschiedenen Druckintervallen durchgeführt. Dadurch ergeben sich zusätzliche Messwerte, die in den Frequenzspektren berücksichtigt werden. Insbesondere kann so der alveoläre Druck bzw. dessen Verlauf in Abhängigkeit von dem im Atemweg vorliegenden Druck genauer bestimmt werden.

Insbesondere wird das Druckintervall so eingestellt, dass eine Normoventilation in diesem Druckintervall möglich ist.

Eine Normoventilation umfasst eine Ventilation, mit der ein Patient für eine zeitlich nicht begrenzte Zeit beatmet werden kann. Es werden also Fluidströme- bzw. volumina zu- und abgeführt, durch die der Patient dauerhaft ausreichend beatmet ist.

Bevorzugt ist, dass das Druckintervall zumindest für ein Zeitintervall reduziert wird, wobei das Verfahren innerhalb dieses Zeitintervalls durchgeführt wird.

Insbesondere werden die Schritte a) bis c) wiederholt durchgeführt, d. h. es werden nacheinander mehrere unterschiedliche Druckintervalle festgelegt, in denen dann gemäß Schritt c) Werte für Druck und Fluidströme erfasst werden. Diese Werte aus unterschiedlichen Druckintervallen werden dann in den Schritten d) bis f) weiterverarbeitet.

Insbesondere umfasst das Druckintervall höchstens 10 mbar, insbesondere höchstens 5 mbar, bevorzugt höchstens 2 mbar. Insbesondere umfasst das Druckintervall mindestens 1 mbar, bevorzugt mindestens 2 mbar.

Insbesondere beträgt das innerhalb des Druckintervalls (und innerhalb des einen Beatmungsvorgangs) zu- und / oder abgeführte Fluidvolumen höchstens 10 %, bevorzugt höchstens 5 %, besonders bevorzugt 2 %, eines maximalen Volumens des Atemwegs. Insbesondere beträgt das Fluidvolumen mindestens 1 %, bevorzugt mindestens 2 % des maximalen Volumens des Atemwegs.

Beträgt also ein maximales Volumen (größtes Volumen eines Atemwegs, ohne dass dieser durch die Ausdehnung geschädigt wird) 2.000 ml wird hier ein Fluidvolumen von höchstens 200 ml zu- und / oder abgeführt.

Insbesondere werden in Schritt b) mindestens fünf, bevorzugt mindestens sieben, besonders bevorzugt mindestens 10 Inspirationsvorgänge und Exspirationsvorgänge durchgeführt.

Insbesondere werden in Schritt c) Werte für den Druck und den Fluidstrom zu jeweils gleichen Zeitpunkten erfasst werden und die Zeitpunkte weisen zeitliche Abstände von höchstens 0,1 Sekunden, bevorzugt von höchstens 0,05 Sekunden, besonders bevorzugt von höchstens 0,01 Sekunden, auf. Insbesondere weisen die Zeitpunkte zeitliche Abstände von mindestens 0,005 Sekunden, bevorzugt von mindestens 0,01 Sekunden auf.

Insbesondere ist die Beatmungsvorrichtung zur ausschließlichen Beatmung des Patienten geeignet ausgeführt; wobei über die Steuereinrichtung zumindest zeitlich vor Schritt a) oder nach Schritt c) eine Normoventilation des Patienten durchführbar ist.

Insbesondere weist die Beatmungsvorrichtung eine Saugeinrichtung auf, so dass in Schritt b) der zweite Fluidstrom in zumindest einem Exspirationsvorgang durch ein Absaugen zumindest teilweise erzeugt wird.

Insbesondere erfolgt bei bekannten Beatmungsvorrichtungen eine passive Exspiration, so dass ein zweiter Fluidstrom ggf. nicht genau erfassbar ist. Insbesondere ist die vorliegende Beatmungsvorrichtung so ausgeführt, dass zu jedem Zeitpunkt die Fluidströme genau bestimmbar und / oder durch die Beatmungsvorrichtung einstellbar sind.

Mittels einer Saugeinrichtung kann insbesondere sichergestellt werden, dass auch zum Ende eines Exspirationsvorgangs hin, also bis zum Erreichen z. B. eines PEEP, ein über den gesamten Exspirationsvorgang konstanter Fluidstrom aufrechterhalten werden kann.

Der Fluidstrom ist zumindest während eines Inspirationsvorgangs und eines Exspirationsvorgangs auf einen konstanten Wert einstellbar, wobei während Schritt b) der erste Fluidstrom $Q_1$ und der zweite Fluidstrom $Q_2$ jeweils konstant sind.

Insbesondere variieren die jeweils eingestellten Fluidströme um höchstens 30 %, bevorzugt um höchstens 20 %, besonders bevorzugt um höchstens 10 %. Ganz besonders bevorzugt variieren die Fluidströme jeweils nur um höchstens 5 % oder sind sogar konstant.

Konstant heißt insbesondere, dass die Fluidströme um weniger als 5 %, bevorzugt um weniger als 1 %, variieren.

Insbesondere erfolgt die Beatmung gemäß Schritt b) ausschließlich mit konstanten Fluidströmen. Insbesondere sind zwischen dem Inspirationsvorgang und dem Exspirationsvorgang keine Pausen vorgesehen, d. h., dass sich die Inspirationsvorgänge und Exspirationsvorgänge unmittelbar aneinander anschließen. Insbesondere sind die Fluidströme gleich groß, d. h. der (inspiratorische) erste Fluidstrom und der (exspiratorische) zweite Fluidstrom sind betragsgleich.

Die Einstellung der Fluidströme, insbesondere auf konstante Fluidströme, erhöht die Genauigkeit des durch die Steuereinrichtung durchgeführten Verfahrens. Insbesondere können so geeignete Frequenzspektren erzeugt werden, aus denen die Impedanz mit hoher Präzision bestimmbar ist. Die Nachbildung der Anteile der Impedanz durch die mathematischen Modelle kann damit mit hoher Genauigkeit erfolgen, so dass die durch die Steuereinrichtung und das darin durchgeführte Verfahren e bestimmbaren Parameter mit hoher Genauigkeit errechnet werden können.

Für eine möglichst präzise Ermittlung der genannten Kennwerte bzw. Parameter (also z. B. des alveolären Druckes, der atemwegsrelatierten sowie der gewebsrelatierten Resistance usw.) ist insbesondere Folgendes nötig bzw. sinnvoll:

    i. stabiler bzw. konstanter, insbesondere vom Betrag gleicher Gasfluss (Fluidstrom) während der Inspiration (Inspirationsvorgang) und Exspiration (Exspirationsvorgang);
    ii. idealerweise tracheale (in der Luftröhre) Messung des Druckes während der Inspiration;
    iii. idealerweise tracheale (in der Luftröhre) Messung des Druckes während der Exspiration.

Gerade bei bestimmten Beatmungsbedingungen (konstanter und gleicher Fluidstrom während Inspiration und Exspiration; Verhältnis 1:1 zwischen Inspiration und Exspiration) ist der alveoläre Druck in geringstem Maße abhängig von dem imaginären Anteil der Impedanz, so dass der alveoläre Druck mit hoher Genauigkeit nur aus dem realen Anteil der Impedanz bestimmbar ist.

Eine flusskontrollierte Ventilation ("Flow-Controlled Ventilation", FCV; z. B. DE 10 2016 103 678.1 und DE 10 2016 109 528.1) ist ein mittlerweile auch klinisch implementierter Beatmungsmodus, bei dem (im Gegensatz zu herkömmlichen Beatmungsvorrichtungen) der Gasfluss nicht nur inspiratorisch, sondern auch exspiratorisch kontrolliert und geregelt wird. Bei FCV entspricht der exspiratorische Gasabfluss insbesondere dem inspiratorischen Gaszufluss; dabei ergibt sich ein Verhältnis von In- und Exspiration von vorzugsweise 1:1. Der Gasfluss (Fluidstrom) ist stabil bzw. konstant (ändert sich also vom Betrag nicht relevant) und dabei vorzugsweise gerade so hoch, dass eine Normoventilation beim Patienten erreicht werden kann. Insbesondere zu Beginn der Exspiration, also ausgehend vom inspiratorischen Spitzendruck, wird der zweite Fluidstrom vorzugsweise reduziert (z. B. durch einen Widerstand). Während der Exspiration und insbesondere gegen Ende der Exspiration, also hin zum endexspiratorischen Druck, wird der zweite Fluidstrom dann zunehmend unterstützt (z. B. durch Saugwirkung).

Es ist nur ein weiteres, experimentelles (klinisch aber bislang nicht verfügbares) Beatmungsverfahren bekannt, bei dem der exspiratorische Gasabfluss moduliert werden kann: Bei "FLow-controlled EXpiration" (FLEX; s. Minerva Anestesiologica 80 (1): 19-28 (2014)) wird eine gewisse Kontrolle der Exspiration durch einen passiven, dynamischen Resistor erreicht, der im Ausatmungsschenkel eines herkömmlichen Beatmungsgerätes angeordnet ist und dessen Widerstand während der Exspiration sukzessiv erniedrigt wird. Abhängig von den Rückstellkräften des Brustkorb-Lunge-Systems und der exspiratorischen (Gesamt-)Resistance kann dieses System den Gasabfluss zwar modulieren, nicht aber einen (weitgehend) stabilen exspiratorischen Gasabfluss schaffen und halten. Auch ein I:E-Verhältnis von 1:1 und somit ein vom Betrag gleicher in- und exspiratorischer Gasfluss (Fluidstrom) ist nicht möglich.

Im Vergleich zu FLEX besteht der Vorteil von FCV darin, dass der exspiratorische Fluidstrom aktiv geregelt (im Sinne eines stabilen bzw. konstanten Gasflusses) und damit bekannt bzw. jederzeit einstellbar ist. Dies kann z. B. mit einem aktiven, dynamischen Resistor erreicht werden (z. B. Kombination eines Widerstandelementes mit einer Saugung, z. B. durch ein Gasstromumkehrelement, z. B. bekannt aus DE 10 2007 013 385.7). Dabei wird z. B. in einer (zeitlich) ersten Hälfte der Exspiration der durch die Rückstellkräfte des Brustkorb-Lunge-Systems besonders hohe Fluidstrom zunächst durch ein Widerstandselement verringert. In einer (zeitlich) zweiten Hälfte der Exspiration, wenn die Rückstellkräfte geringer werden und damit der Fluidstrom üblicherweise sukzessive abnehmen würde, wird der Fluidstrom von einer Saugung (z. B. einem Unterdruckanschluss oder ähnlichem) erhöht und insgesamt konstant gehalten.

Insbesondere in der (zeitlich) zweiten Hälfte der Exspiration ist der Gasabfluss (zweiter Fluidstrom) damit sehr stabil und kann vom Betrag insbesondere dem inspiratorischen Gaszufluss (erstem Fluidstrom) entsprechend eingestellt werden, also insbesondere über den gesamten Exspirationsvorgang hinweg.

Verglichen mit Beatmungsverfahren, die mit in- und / oder exspiratorisch dezelerierendem Gasfluss arbeiten (z. B. VCV, also volumenkontrollierte Beatmung mit nur inspiratorisch geregeltem Gasfluss, oder PCV, also druckkontrollierte Beatmung mit ebenfalls nur inspiratorisch geregeltem Gasfluss), schafft FCV optimale Bedingungen für die hier beschriebenen Messungen und Berechnungen.

Für eine möglichst genaue Berechnung des (globalen) alveolären Druckes bzw. Druckverlaufes ist ein jeweils stabiler bzw. konstanter, insbesondere vom Betrag gleicher, in- und exspiratorischer Fluidstrom bevorzugt. Diese Voraussetzungen erfüllt nur FCV.

Auch aus anderen (z. B. mechanischen und energetischen) Gründen ist eine Beatmung mit langsamen, gleichmäßigen Druck- und Volumenänderungen unter Nutzung des Bereiches individuell optimaler, also maximaler, Compliance sinnvoll.

FCV ist insbesondere für die kontrollierte, maximal lungenprotektive Beatmung, nicht aber zur Unterstützung von Spontanatmung gedacht, da hierfür deutlich höhere Gasflüsse notwendig sind.

Insbesondere durch einen möglichst niedrigen, stabilen und vom Betrag in- und exspiratorisch gleichen Fluidstrom können Unterschiede hinsichtlich der Gasverteilung in der Lunge (innerhalb des physikalisch Möglichen) minimiert werden. CT-Untersuchungen und elektrische Impedanz-Tomografie haben eine insgesamt bessere und homogenere Belüftung sowohl gesunder als auch kranker Lungen durch FCV bereits belegt.

Ziel der Erfindung ist es also insbesondere, eine Beatmungsvorrichtung zu beschreiben, die bzw. das eine (im Rahmen des physikalisch Möglichen) genaue Bestimmung von Kennwerten bzw. Parametern (z. B. die Ermittlung des (globalen) alveolären Druckes bzw. Druckverlaufes sowie der atemwegs- und gewebsrelatierten Resistance) während der kontrollierten Beatmung eines Patienten ermöglicht.

Bei der Beatmungsvorrichtung handelt es sich insbesondere um ein Beatmungsgerät, das mit einem kontinuierlichen (ohne relevante Pausen), jeweils stabilen bzw. konstantem, vom Betrag in- und exspiratorisch gleichen Fluidstrom (und damit einem I:E-Verhältnis von typischerweise 1:1) vorzugsweise im Bereich optimaler bzw. maximaler Compliance beatmet. Dabei ist der Fluidstrom gerade so hoch, dass Normoventilation bzw. der gewünschte Grad der Kohlendioxidelimination bzw. -abatmung beim Patienten erreicht wird.

Ein Anwender oder vorzugsweise eine (automatisch arbeitende) Steuereinrichtung der Beatmungsvorrichtung kann, unter Berücksichtigung eines ermittelten oder zusätzlich abgeschätzten Verlaufs zumindest eines Teilbereichs einer Compliance-Kurve in einem Druck-Volumen-Diagramm, eine Lage eines Druckintervalls mit den Drücken $P_I$ und $P_E$ bestimmen und diese Drücke an der Beatmungsvorrichtung einstellen (z. B. PIP als $P_I$ bzw. als ersten Druck und PEEP als $P_E$ bzw. als dritten Druck), so dass zumindest ein Beatmungsvorgang, also eine Inspiration und / oder eine Exspiration, zwischen diesen Drücken $P_I$ und $P_E$ erfolgt und dadurch über das resultierende $V_T$ ein Betrag der Compliance dieses Beatmungsvorgangs möglichst groß ist. Alternativ kann die Lage eines Druckintervalls mit den Drücken $P_I$ und $P_E$ auch in einem Volumen-Druck-Diagramm ermittelt werden. Der Beatmungsvorgang sollte zudem so eingestellt werden, dass ein für eine Normoventilation erforderliches Minutenvolumen bei möglichst maximaler Compliance zu- und abgeführt werden kann, da ein (optimal) großes $V_T$ mit einer (optimal) niedrigen Beatmungsfrequenz die Effizienz der Kohlendioxidelimination erhöht und so der Atemweg bzw. das Gewebe des Patienten möglichst wenig belastet wird.

Auch wenn der in- und exspiratorische Fluidstrom vom Betrag unterschiedlich sein können, ist insbesondere nur eine Abweichung des aktuell vorliegenden Fluidstroms um höchstens 10 %, bevorzugt höchstens 5 %, besonders bevorzugt höchstens 1 %, jeweils während des Inspirationsvorgangs und während des Exspirationsvorgangs von einem eingestellten oder durchschnittlich vorliegenden Fluidstrom vorgesehen. Insbesondere ist eine entsprechende Abweichung auch zwischen Inspirationsvorgang und Exspirationsvorgang möglich. Insbesondere beträgt aber das Verhältnis zwischen Inspirationsvorgang und Exspirationsvorgang 1:1, ist also der Fluidstrom konstant und betragsgleich für den Inspirationsvorgang und den Exspirationsvorgang.

Mit der Beatmungsvorrichtung können, ausgehend von den gemessenen Werten des Druckes und dem (ggf. aktiv gesteuerten und damit zu jedem Zeitpunkt bekannten) in- bzw. exspiratorischen Fluidstrom, die genannten Kennwerte bzw. Parameter (atemwegs- und gewebsrelatierten Resistance und (globaler) alveolärer Druck bzw. Druckverlauf) ermittelt und wahlweise ausgegeben werden (z. B. auf einem Display der Beatmungsvorrichtung).

Es wird weiter ein Verfahren zur Bestimmung zumindest eines alveolären Druckes bzw. eines Verlaufs eines alveolären Druckes eines Patienten mit einer Beatmungsvorrichtung beschrieben insbesondere mit der beschriebenen Beatmungsvorrichtung.

Die Beatmungsvorrichtung umfasst zumindest eine Gaszuführeinrichtung und eine Gasabführeinrichtung, zum Zuführen eines (inspiratorischen) ersten Fluidstroms hin zu einem Atemweg eines Patienten und zum Abführen eines (exspiratorischen) zweiten Fluidstroms aus dem Atemweg (des Patienten) zurück in die Beatmungsvorrichtung oder an eine Umgebung, einen Drucksensor zur Erfassung eines Druckes in dem Atemweg sowie eine Steuereinrichtung zum Betreiben der Beatmungsvorrichtung. Die Steuereinrichtung ist zur Durchführung des Verfahrens eingerichtet, das zumindest die folgenden Schritte umfasst:

a) Festlegen eines Druckintervalls, in dem der Patient für ein festgelegtes Zeitintervall zu beatmen ist;
b) wiederholtes abwechselndes Durchführen von jeweils einem Inspirationsvorgang mit einem ersten Fluidstrom $Q_1$ mittels der Gaszuführeinrichtung und von jeweils einem Exspirationsvorgangs mit einem zweiten Fluidstrom $Q_2$ mittels der Gasabführeinrichtung innerhalb des Druckintervalls,
c) (Messen oder Bestimmen oder) Erfassen des sich während Schritt b) verändernden Druckes und der Fluidströme;
d) Durchführen einer Fouriertransformation für die erfassten Werte des Druckes und Bilden eines ersten Frequenzspektrums für den Druck sowie Durchführen einer Fouriertransformation für die erfassten Werte der Fluidströme und Bilden eines zweiten Frequenzspektrums für die Fluidströme;
e) Berechnen einer Impedanz $Z_{aw}$ des Atemwegs durch Dividieren des ersten Frequenzspektrums durch das zweite Frequenzspektrum, wobei die Impedanz einen realen Anteil $Real(Z_{aw})$ und einen imaginären Anteil $Im(Z_{aw})$ aufweist;
f) Nachbilden zumindest des realen Anteils durch ein mathematisches erstes Modell und Ermitteln eines alveolären Druckes bzw. eines Verlaufs eines alveolären Druckes.

Die obige (nicht abschließende) Einteilung der Verfahrensschritte in a) bis f) soll vorrangig nur zur Unterscheidung dienen und keine Reihenfolge und / oder Abhängigkeit erzwingen. Auch die Häufigkeit der Verfahrensschritte z. B. während der Einrichtung und / oder des Betriebes der Beatmungsvorrichtung kann variieren. Ebenso ist möglich, dass Verfahrensschritte einander zumindest teilweise zeitlich überlagern. Ganz besonders bevorzugt finden die Verfahrensschritte a) bis c) jeweils nacheinander statt. Es ist aber auch möglich die Verfahrensschritte a) bis c) mehrmals zu wiederholen (also z. B. für unterschiedliche Druckintervalle). Schritt d) kann insbesondere nach der einmaligen Durchführung der Schritte a) bis c) oder auch nach einer mehrmaligen Durchführung der Schritte a) bis c) durchgeführt werden. Schritte e) und f) werden insbesondere nach Schritt d) durchgeführt. Insbesondere werden die Schritte a) bis f) in der angeführten Reihenfolge durchgeführt.

Insbesondere ist die Beatmungsvorrichtung zur ausschließlichen Beatmung des Patienten geeignet ausgeführt, wobei über die Steuereinrichtung zumindest zeitlich vor Schritt a) eine Normoventilation des Patienten durchgeführt wird.

Insbesondere weist die Beatmungsvorrichtung eine Saugeinrichtung auf, so dass in Schritt b) der zweite Fluidstrom in zumindest einem Exspirationsvorgang durch ein Absaugen zumindest teilweise erzeugt wird.

Der Fluidstrom ist zumindest während eines Inspirationsvorgangs und eines Exspirationsvorgangs auf einen konstanten Wert eingestellt; wobei während Schritt b) der erste Fluidstrom $Q_1$ und der zweite Fluidstrom $Q_2$ jeweils konstant sind.

Es wird weiter eine Steuereinrichtung für eine Beatmungsvorrichtung, insbesondere für die beschriebene Beatmungsvorrichtung, beschrieben, die zur Durchführung des beschriebenen Verfahrens (geeignet) ausgestattet, konfiguriert oder programmiert ist.

Die Ausführungen zur Beatmungsvorrichtung sind insbesondere auf das beschriebene Verfahren und die beschriebene Steuereinrichtung übertragbar und jeweils umgekehrt

Weiter kann das beschriebene Verfahren auch von einem Anwender (teilweise) manuell oder einem (separaten) Computer bzw. mit einem Prozessor einer Steuereinrichtung halbautomatisch oder (voll)automatisch ausgeführt werden.

Es wird demnach auch ein System zur Datenverarbeitung beschrieben, das einen Prozessor umfasst, der so angepasst, programmiert und konfiguriert ist, dass er das beschriebene Verfahren bzw. einen Teil der Schritte des Verfahrens (ggf. im Dialog mit einem Anwender) durchführt.

Es kann ein computerlesbares Speichermedium vorgesehen sein, das Befehle / Algorithmen umfasst, die bei der Ausführung durch einen Computer / Prozessor diesen veranlassen, das beschriebene Verfahren bzw. mindestens einen Teil der Schritte des Verfahrens (ggf. im Dialog mit einem Anwender) auszuführen.

Die Verwendung unbestimmter Artikel ("ein", "eine", "einer" und "eines"), insbesondere in den Patentansprüchen und in der diese wiedergebenden Beschreibung, ist als solche und nicht als Zahlwort zu verstehen. Entsprechend damit eingeführte Begriffe bzw. Komponenten sind somit so zu verstehen, dass diese mindestens einmal vorhanden sind und insbesondere aber auch mehrfach vorhanden sein können.

Vorsorglich sei angemerkt, dass die hier verwendeten Zahlwörter ("erste", "zweite", ...) vorrangig (nur) zur Unterscheidung von mehreren gleichartigen Gegenständen, Größen oder Prozessen dienen, also insbesondere keine Abhängigkeit und / oder Reihenfolge dieser Gegenstände, Größen oder Prozesse zueinander zwingend vorgeben. Sollte eine Abhängigkeit und / oder Reihenfolge erforderlich sein, ist dies hier explizit angegeben oder es ergibt sich offensichtlich für den Fachmann beim Studium der konkret beschriebenen Ausgestaltung. Soweit ein Bauteil mehrfach vorkommen kann ("mindestens ein"), kann die Beschreibung zu einem dieser Bauteile für alle oder ein Teil der Mehrzahl dieser Bauteile gleichermaßen gelten; dies ist aber nicht zwingend.

Die Erfindung sowie das technische Umfeld werden nachfolgend anhand der beiliegenden Figuren näher erläutert. Es ist darauf hinzuweisen, dass die Erfindung durch die angeführten Ausführungsbeispiele nicht beschränkt werden soll. Insbesondere ist es, soweit nicht explizit anders dargestellt, auch möglich, Teilaspekte der in den Figuren erläuterten Sachverhalte zu extrahieren und mit anderen Bestandteilen und Erkenntnissen aus der vorliegenden Beschreibung zu kombinieren. Insbesondere ist darauf hinzuweisen, dass die Figuren und insbesondere die dargestellten Größenverhältnisse nur schematisch sind. Es zeigen:

Fig. 1: eine Beatmungsvorrichtung im Betrieb;

Fig. 2: ein Diagramm mit einem Verlauf eines Druckes während eines Beatmungsvorgangs;

Fig. 3: ein Diagramm mit einem Verlauf eines Fluidstroms während des Beatmungsvorgangs nach Fig. 2;

Fig. 4: ein Diagramm mit Frequenzspektren des Beatmungsvorgangs nach Fig. 2 und 3;

Fig. 5: ein Diagramm mit den Anteilen der komplexen Impedanz der Frequenzspektren nach Fig. 4; und

Fig. 6: ein Diagramm mit dem Verlauf eines Druckes während der Durchführung des Verfahrens.

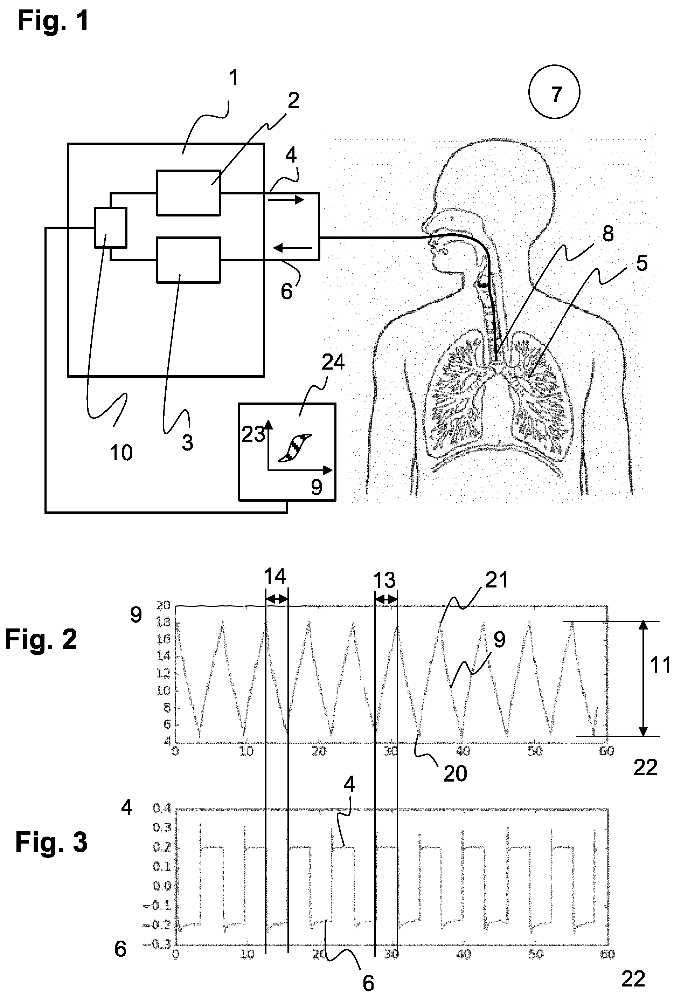

Die Fig. 1 zeigt eine Beatmungsvorrichtung 1 im Betrieb. Die Beatmungsvorrichtung 1 umfasst eine Gaszuführeinrichtung 2 und eine Gasabführeinrichtung 3, zum Zuführen eines (inspiratorischen) ersten Fluidstroms 4 hin zu einem Atemweg 5 eines Patienten und zum Abführen eines (exspiratorischen) zweiten Fluidstroms 6 aus dem Atemweg 5 zurück in die Beatmungsvorrichtung 1 oder in die Umgebung 7, einen Drucksensor 8 zur Erfassung eines Druckes 9 in dem Atemweg 5 sowie eine Steuereinrichtung 10 zum Betreiben der Beatmungsvorrichtung 1. Der Fluidstrom 4, 6 kann während eines Inspirationsvorgangs 13 und eines Exspirationsvorgangs 14 auf einen konstanten Wert eingestellt werden. Die Steuereinrichtung 10 ist zum Betreiben der Beatmungsvorrichtung 1 und zur Durchführung des Messverfahrens geeignet ausgeführt.

Der Drucksensor 8 ist endotracheal angeordnet. Der Drucksensor 8 befindet sich am distalen Ende eines Beatmungskatheters, der als Bestandteil der Beatmungsvorrichtung 1 in dem Atemweg 5 des Patienten angeordnet ist.

Die Beatmungsvorrichtung 1 umfasst weiter eine Visualisierungseinrichtung 24 (z. B. ein Display), auf der die (Gesamt-)Resistance, atemwegsrelatierte Resistance und gewebsrelatierte Resistance, insbesondere aber auch der aktuelle alveoläre Druck 9 über die Zeit 22 und / oder der Verlauf des alveolären Druckes 9 und Volumens 23 (als Druck-Volumen-Kurve) darstellbar sind.

Fig. 2 zeigt ein Diagramm mit einem Verlauf eines Druckes 9 während eines Beatmungsvorgangs. Fig. 3 zeigt ein Diagramm mit einem Verlauf eines Fluidstroms 4, 6 während des Beatmungsvorgangs nach Fig. 2. Die Fig. 2 und 3 werden im Folgenden gemeinsam beschrieben. Auf die Ausführungen zu Fig. 1 wird verwiesen.

Auf der vertikalen Achse des Diagramms nach Fig. 2 ist der Druck 9 in [mbar] aufgetragen. Auf der vertikalen Achse des Diagramms nach Fig. 3 sind die Fluidströme 4, 6 in [Liter / Sekunde] aufgetragen. Auf den horizontalen Achsen der Diagramme ist die Zeit 22 aufgetragen.

Gemäß Schritt a) des Verfahrens erfolgt ein Festlegen eines Druckintervalls 11, in dem der Patient für ein festgelegtes Zeitintervall 12 (hier nicht festgelegt) zu beatmen ist. Gemäß Schritt b) erfolgt ein wiederholtes abwechselndes Durchführen von jeweils einem Inspirationsvorgang 13 mit einem ersten Fluidstrom $Q_1$ 4 mittels der Gaszuführeinrichtung 2 und von jeweils einem Exspirationsvorgangs 14 mit einem zweiten Fluidstrom $Q_2$ 6 mittels der Gasabführeinrichtung 3 innerhalb des Druckintervalls 11. Gemäß Schritt c) erfolgt ein Erfassen des sich während Schritt b) verändernden Druckes 9 und der Fluidströme 4, 6.

Die Beatmung erfolgt kontinuierlich (also ohne relevante Pausen) mit jeweils stabilen bzw. konstantem, vom Betrag in- und exspiratorisch gleichen Fluidströmen 4, 6 (und damit einem I:E-Verhältnis von typischerweise 1:1) vorzugsweise im Bereich optimaler bzw. maximaler Compliance. Dabei ist der Fluidstrom 4, 6 gerade so hoch, dass Normoventilation bzw. der gewünschte Grad der Kohlendioxidelimination bzw. -abatmung beim Patienten erreicht wird.

Es ist erkennbar, dass eine Beatmungsfrequenz von ca. 0,167 Hz vorliegt, d. h. es werden fünf Beatmungsvorgänge in 30 Sekunden durchgeführt.

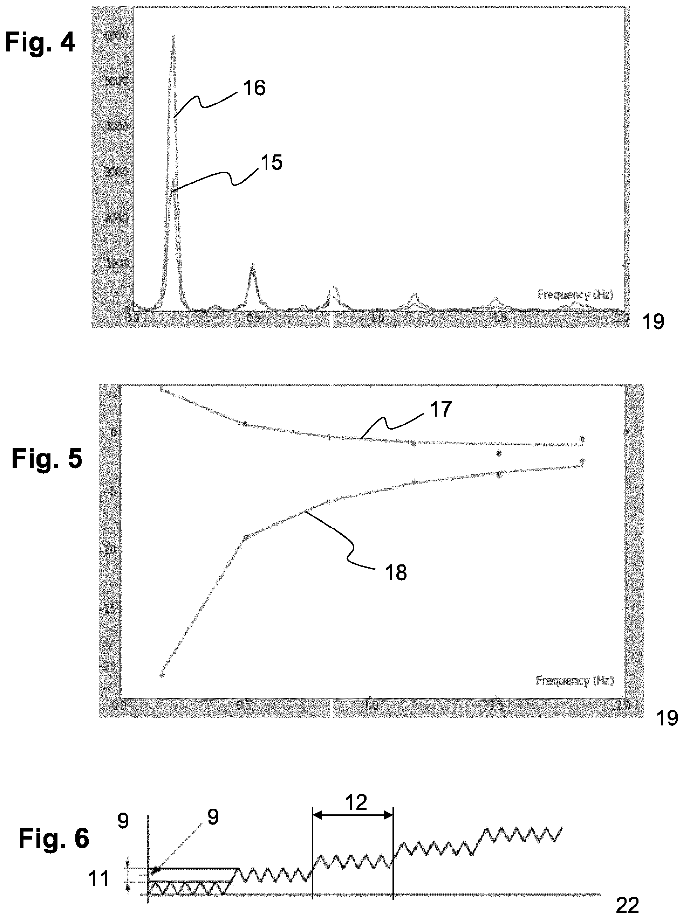

Fig. 4 zeigt ein Diagramm mit Frequenzspektren 15, 16 des Beatmungsvorgangs nach Fig. 2 und 3. Fig. 5 zeigt ein Diagramm mit den Anteilen 17, 18 der komplexen Impedanz der Frequenzspektren 15, 16 nach Fig. 4. Auf die Ausführungen zu den Fig. 1 bis 3 wird verwiesen.

Gemäß Schritt d) erfolgt ein Durchführen einer Fouriertransformation für die erfassten Werte des Druckes 9 und Bilden eines ersten Frequenzspektrums 15 für den Druck 9 sowie Durchführen einer Fouriertransformation für die erfassten Werte der Fluidströme 4, 6 und Bilden eines zweiten Frequenzspektrums 16 für die Fluidströme 4, 6. Es ist erkennbar, dass die Frequenzspektren 15, 16 klar erkennbare lokale Maxima aufweisen, z. B. bei der Beatmungs-Frequenz 19, also bei 0,167 Hz, und bei Vielfachem der Beatmungs-Frequenz 19, also bei 3 x Beatmungs-Frequenz 19, bei 5 x Beatmungs-Frequenz, bei 7 x Beatmungs-Frequenz 19, bei 9 x Beatmungs-Frequenz 19, usw.

Gemäß Schritt e) erfolgt ein Berechnen einer Impedanz $Z_{aw}$ des Atemwegs 5 durch Dividieren des ersten Frequenzspektrums 15 durch das zweite Frequenzspektrum 16, wobei die Impedanz einen realen Anteil Real($Z_{aw}$) 17 und einen imaginären Anteil Im($Z_{aw}$) 18 aufweist.

Die Impedanz wird jeweils für die, die lokalen Maxima erzeugenden, Frequenzen 19 ermittelt. In Fig. 5 sind die Werte der einzelnen Anteile 17, 18 für die jeweilige Frequenz 19 dargestellt. Die einzelnen so ermittelten Werte der Anteile 17, 18 der Impedanz, bzw. Punkte im Diagramm, können dann gemäß Schritt f) durch eine Kurve, also durch ein mathematisches erstes Modell, approximiert bzw. nachgebildet werden.

Gemäß Schritt f) erfolgt also ein Nachbilden zumindest des realen Anteils 17 durch ein mathematisches erstes Modell und Ermitteln eines alveolären Druckes 9 bzw. eines Verlaufs eines alveolären Druckes 9.

Das erste Modell umfasst die Gleichung

$$Real(Z_{aw}) = R_{aw} + \frac{G}{\omega^\alpha} \text{ , mit}$$

$R_{aw}$: atemwegsrelatierte Resistance;

$\frac{G}{\omega^\alpha}$ : gewebsrelatierte Resistance; mit G als einer Konstanten, $\omega$ der Kreisfrequenz (also 2 x $\pi$ x Frequenz der Beatmung) und $\alpha$ als einer Konstanten; wobei der reale Anteil 17 die Resistance, also die während der In- bzw. Exspiration zu überwindenden Widerstände des Atemwegs 5, beschreibt.

Der alveoläre Druck 9 $P_{alv}$ bzw. dessen Verlauf wird aus der Gleichung $P_{alv} = P_{trach} - Q_i \times R_{aw}$ ermittelt, mit

$Q_i$: dem jeweils während Schritt b) vorliegenden Fluidstrom 4, 6 (siehe Fig. 3).

Der gemessene oder bestimmte Druck 9 $P_{trach}$ ist der über die Zeit 22 sich verändernde Druck 9 in dem Atemweg 5 (siehe Fig. 2). Der Verlauf des Druckes 9 $P_{alv}$ über die Zeit 22 ergibt sich also in Abhängigkeit von dem sich über die Zeit 22 ändernden Druck 9 $P_{trach}$.

Insbesondere ist in Schritt f) durch ein mathematisches zweites Modell auch der imaginäre Anteil 18 (siehe Fig. 5) nachbildbar, wobei das zweite Modell die Gleichung

$$Z_{aw} = R_{aw} + k \times j \times \omega \times I_{aw} + \frac{G - j \times H}{\omega^\alpha}$$

umfasst; wobei

$$Im(Z_{aw}) = j \times (k \times \omega \times I_{aw} + \frac{-H}{\omega^\alpha} ;$$

mit

k: einer Konstanten;

$l_{aw}$: Trägheit des Atemwegs 5;

$\frac{-H}{\omega^\alpha}$ : Elastizität des Atemwegs 5 mit H als einer Konstanten;

wobei der imaginäre Anteil 18 die Atemwegsreactance $X_a$ beschreibt, wobei eine Compliance des Atemwegs 5 durch $C = -\frac{1}{\omega \times X_a}$ beschrieben ist.

Der imaginäre Anteil 18 ist für die Bestimmung des alveolären Druckes 9 bzw. dessen Verlaufs nicht zu ermitteln. Allerdings können aus dem imaginären Anteil 18 weitere ggf. als relevant erachtete Parameter abgeleitet werden, z. D. die Atemwegsreactance und die Compliance des Atemwegs 5.

Fig. 6 zeigt ein Diagramm mit dem Verlauf eines Druckes 9 während der Durchführung des Verfahrens. Auf der vertikalen Achse des Diagramms ist der Druck 9 in [mbar] aufgetragen. Auf der horizontalen Achse ist die Zeit 22 aufgetragen.

Auf die Ausführungen zu Fig. 1 bis 5 wird verwiesen.

Bevorzugt ist, dass das Druckintervall 11 zumindest für ein Zeitintervall 12 reduziert wird, wobei das Verfahren innerhalb dieses Zeitintervalls 12 durchgeführt wird.

Die Schritte a) bis c) werden hier wiederholt durchgeführt, d. h. es werden nacheinander mehrere unterschiedliche Druckintervalle 11 festgelegt, in denen dann gemäß Schritt c) Werte für Druck 9 und Fluidströme 4, 6 erfasst werden. Diese Werte aus unterschiedlichen Druckintervallen 11 werden dann in den Schritten d) bis f) weiterverarbeitet.

In jedem Schritt b) werden fünf Inspirationsvorgänge 13 und Exspirationsvorgänge 14 (vgl. Fig. 2) durchgeführt.

Jedes Druckintervall 11 wird einem mittleren Druck 9 zugeordnet, wobei das Druckintervall 11 in einem endexspiratorischen Zustand 20 durch einen Druck 9 PEEP (positive end expiratory pressure) und in einem endinspiratorischem Zustand 21 durch einen Druck 9 PIP (peak inspiratory pressure) begrenzt ist.

Das Druckintervall 11 umfasst z. B. höchstens 5 mbar, wobei mit jedem Beatmungsvorgang nur ein geringes Volumen 23 des Fluids zu- bzw. abgeführt wird. Z. B. beträgt das innerhalb des Druckintervalls 11 und innerhalb des einen Beatmungsvorgangs zu- und / oder abgeführte Volumen 23 des Fluids höchstens 10 % eines maximalen Volumens des Atemwegs 5.

Hier werden nacheinander fünf Druckintervalle 11 festgelegt, wobei die Beatmung des Patienten mit der Beatmungsvorrichtung 1 erfolgt und der Atemweg 5 mit den unterschiedlichen Druckintervallen unmittelbar nacheinander beaufschlagt wird.

## Bezugszeichenliste

| 1 | Beatmungsvorrichtung |
|---|---|
| 2 | Gaszuführeinrichtung |
| 3 | Gasabführeinrichtung |
| 4 | erster Fluidstrom |
| 5 | Atemweg |
| 6 | zweiter Fluidstrom |
| 7 | Umgebung |
| 8 | Drucksensor |
| 9 | Druck |
| 10 | Steuereinrichtung |
| 11 | Druckintervall |
| 12 | Zeitintervall |
| 13 | Inspirationsvorgang |
| 14 | Exspirationsvorgang |
| 15 | erstes Frequenzspektrum |
| 16 | zweites Frequenzspektrum |
| 17 | realer Anteil |
| 18 | imaginärer Anteil |
| 19 | Frequenz |
| 20 | endexspiratorischer Zustand |
| 21 | endinspiratorischer Zustand |
| 22 | Zeit |
| 23 | Volumen |
| 24 | Visualisierungseinrichtung |

## Patentansprüche

1. Beatmungsvorrichtung (1), zumindest umfassend eine Gaszuführeinrichtung (2) und eine Gasabführeinrichtung (3), zum Zuführen eines ersten Fluidstroms (4) hin zu einem Atemweg (5) eines Patienten und zum Abführen eines zweiten Fluidstroms (6) aus dem Atemweg (5) zurück in die Beatmungsvorrichtung (1) oder an eine Umgebung (7), einen Drucksensor (8) zur Erfassung eines Druckes $P_{trach}$ (9) in dem Atemweg (5) sowie eine Steuereinrichtung (10) zum Betreiben der Beatmungsvorrichtung (1); wobei die Steuereinrichtung (10) zur Durchführung eines Verfahrens eingerichtet ist, das zumindest die folgenden Schritte umfasst:

   a) Festlegen eines Druckintervalls (11), in dem der Patient für ein festgelegtes Zeitintervall (12) zu beatmen ist;
   b) wiederholtes abwechselndes Durchführen von jeweils einem Inspirationsvorgang (13) mit dem ersten Fluidstrom (4) $Q_1$ mittels der Gaszuführeinrichtung (2) und von jeweils einem Exspirationsvorgangs (14) mit dem zweiten Fluidstrom (6) $Q_2$ mittels der Gasabführeinrichtung (3) innerhalb des Druckintervalls (11),
   c) Erfassen des sich während Schritt b) verändernden Druckes (9) und der Fluidströme (4, 6);
   d) Durchführen einer Fouriertransformation für die erfassten Werte des Druckes (9) und Bilden eines ersten Frequenzspektrums (15) für den Druck (9) sowie Durchführen einer Fouriertransformation für die erfassten Werte der Fluidströme (4, 6) und Bilden eines zweiten Frequenzspektrums (16) für die Fluidströme (4, 6);

e) Berechnen einer Impedanz $Z_{aw}$ des Atemwegs (5) durch Dividieren des ersten Frequenzspektrums (15) durch das zweite Frequenzspektrum (16), wobei die Impedanz einen realen Anteil Real($Z_{aw}$) (17) und einen imaginären Anteil Im($Z_{aw}$) (18) aufweist;

f) Nachbilden zumindest des realen Anteils (17) durch ein mathematisches erstes Modell und Ermitteln eines alveolären Druckes $P_{alv}$ (9) bzw. eines Verlaufs eines alveolären Druckes $P_{alv}$ (9);

wobei das erste Modell die Gleichung

$$Real(Z_{aw}) = R_{aw} + {}^{G}\!/_{\omega^\alpha}$$ umfasst, mit

$R_{aw}$: atemwegsrelatierte Resistance;

${}^{G}\!/_{\omega^\alpha}$ : gewebsrelatierte Resistance; mit G als einer Konstanten, $\omega$ der Kreisfrequenz und $\alpha$ als einer Konstanten; wobei der reale Anteil (17) die Resistance, also die während der In- bzw. Exspiration zu überwindenden Widerstände, beschreibt;

wobei der alveoläre Druck $P_{alv}$ aus der Gleichung $P_{alv} = P_{trach} - Q_i \times R_{aw}$ ermittelt wird; mit $Q_i$: dem jeweils vorliegenden Fluidstrom (4, 6); **dadurch gekennzeichnet, dass** in Schritt f) der alveoläre Druck $P_{alv}$ (9) bzw. der Verlauf des alveolären Druckes $P_{alv}$ (9) nur anhand des realen Anteils (17) ermittelt wird; wobei der Fluidstrom (4, 6) zumindest während eines Inspirationsvorgangs (13) und eines Exspirationsvorgangs (14) auf einen konstanten Wert einstellbar ist; wobei während Schritt b) der erste Fluidstrom (4) $Q_1$ und der zweite Fluidstrom (6) $Q_2$ jeweils konstant sind.

2. Beatmungsvorrichtung (1) nach dem vorhergehenden Patentanspruch 1, wobei in Schritt f) durch ein mathematisches zweites Modell auch der imaginäre Anteil (18) nachbildbar ist, wobei das zweite Modell die Gleichung

$$Z_{aw} = R_{aw} + k \times j \times \omega \times I_{aw} + \frac{G - j \times H}{\omega^\alpha}$$

umfasst; wobei

$$Im(Z_{aw}) = j \times (k \times \omega \times I_{aw} + \frac{-H}{\omega^\alpha};$$

mit

k: einer Konstanten;

$I_{aw}$: Trägheit des Atemwegs (5);

$\frac{-H}{\omega^\alpha}$ : Elastizität des Atemwegs (5) mit H als einer Konstanten;

wobei der imaginäre Anteil (18) die Atemwegsreactance $X_a$ beschreibt, wobei eine Compliance des Atemwegs (5) durch

$$C = -\frac{1}{\omega \times X_a}$$ beschrieben ist.

3. Beatmungsvorrichtung (1) nach einem der vorhergehenden Patentansprüche, wobei der Drucksensor (8) in Anwendung endotracheal angeordnet ist.

4. Beatmungsvorrichtung (1) nach einem der vorhergehenden Patentansprüche, wobei zumindest die Schritte a) bis c) in verschiedenen Druckintervallen (11) durchgeführt werden.

5. Beatmungsvorrichtung (1) nach einem der vorhergehenden Patentansprüche, wobei das Druckintervall (11) höchstens 10 mbar umfasst.

6. Beatmungsvorrichtung (1) nach einem der vorhergehenden Patentansprüche, wobei das innerhalb des Druckintervalls (11) zu- oder abgeführte Fluidvolumen höchstens 10 % eines maximalen Volumens des Atemwegs (5) beträgt.

7. Beatmungsvorrichtung (1) nach einem der vorhergehenden Patentansprüche, wobei in Schritt b) mindestens fünf Inspirationsvorgänge (13) und Exspirationsvorgänge (14) durchgeführt werden.

8. Beatmungsvorrichtung (1) nach einem der vorhergehenden Patentansprüche, wobei in Schritt c) Werte für den Druck (9) und den Fluidstrom (4, 6) zu jeweils gleichen Zeitpunkten erfasst werden und die Zeitpunkte zeitliche Abstände von höchstens 0,1 Sekunden aufweisen.

9. Beatmungsvorrichtung (1) nach einem der vorhergehenden Patentansprüche, wobei die Beatmungsvorrichtung (1) zur ausschließlichen Beatmung des Patienten geeignet ausgeführt ist; wobei über die Steuereinrichtung (10) zumindest zeitlich vor Schritt a) oder nach Schritt c) eine Normoventilation des Patienten durchführbar ist.

10. Beatmungsvorrichtung (1) nach einem der vorhergehenden Patentansprüche, wobei die Gasabführeinrichtung (3) eine Saugeinrichtung aufweist, so dass in Schritt b) der zweite Fluidstrom (6) in zumindest einem Exspirationsvorgang (14) durch ein Absaugen zumindest teilweise erzeugt wird.

**11.** Beatmungsvorrichtung (1) nach einem der vorhergehenden Patentansprüche, wobei die Fluidströme (4, 6) gleich groß sind.

**Claims**

**1.** Ventilator (1), at least comprising a gas supply device (2) and a gas discharge device (3), for supplying a first fluid flow (4) to an airway (5) of a patient and for discharging a second fluid flow (6) from the airway (5) back into the ventilator (1) or to an environment (7), a pressure sensor (8) for sensing a pressure $P_{trach}$ (9) in the airway (5), and a control device (10) for operating the ventilator (1); wherein the control device (10) is configured to carry out a method comprising at least the following steps:

a) defining a pressure interval (11) in which the patient is to be ventilated for a defined time interval (12);
b) repeatedly and alternately carrying out one inspiration process (13) at a time with the first fluid flow (4) $Q_1$ by means of the gas supply device (2) and one expiration process (14) at a time with the second fluid flow (6) $Q_2$ by means of the gas discharge device (3) within the pressure interval (11),
c) sensing the fluid flows (4, 6) and the pressure (9) which changes during step b);
d) carrying out a Fourier transform for the sensed values of the pressure (9) and forming a first frequency spectrum (15) for the pressure (9) and carrying out a Fourier transform for the sensed values of the fluid flows (4, 6) and forming a second frequency spectrum (16) for the fluid flows (4, 6);
e) calculating an impedance $Z_{aw}$ of the airway (5) by dividing the first frequency spectrum (15) by the second frequency spectrum (16), wherein the impedance comprises a real component $Real(Z_{aw})$ (17) and an imaginary component $Im(Z_{aw})$ (18);
f) modeling at least the real component (17) by a first mathematical model and ascertaining an alveolar pressure $P_{alv}$ (9) or a plot of an alveolar pressure $P_{alv}$ (9);
wherein the first model comprises the equation

$$Real(Z_{aw}) = R_{aw} + {}^G\!/_{\omega^\alpha}, \text{ with}$$

$R_{aw}$: airway-related resistance;

${}^G\!/_{\omega^\alpha}$ : tissue-related resistance; with G as a constant, $\omega$ as the angular frequency and $\alpha$ as a constant; wherein the real component (17) describes the resistance, i.e., the resistances to be overcome during inspiration or expiration;
wherein the alveolar pressure $P_{alv}$ is ascer-

tained from the equation $P_{alv} = P_{trach} - Q_i \times R_{aw}$; with
$Q_i$: the current fluid flow (4, 6);
**characterized in that** in step f) the alveolar pressure $P_{alv}$ (9) or the plot of the alveolar pressure $P_{alv}$ (9) is determined only from the real component;
wherein the fluid flow (4, 6) is adjustable to a constant value at least during an inspiration process (13) and an expiration process (14);
wherein the first fluid flow (4) $Q_1$ and the second fluid flow (6) $Q_2$ are both constant during step b).

**2.** Ventilator (1) as claimed in the preceding claim 1, wherein also the imaginary component (18) is modelable in step f) by a second mathematical model, wherein the second model comprises the equation

$$Z_{aw} = R_{aw} + k \times j \times \omega \times I_{aw} + \frac{G - j \times H}{\omega^\alpha};$$

where

$$Im(Z_{aw}) = j \times (k \times \omega \times I_{aw} + \frac{-H}{\omega^\alpha};$$

with

k: a constant;
$I_{aw}$: inertia of the airway (5);

$\frac{-H}{\omega^\alpha}$ : resilience of the airway (5) with H as a constant;
wherein the imaginary component (18) describes the airway reactance $X_a$,
wherein a compliance of the airway (5) is described by $C = -\frac{1}{\omega \times X_a}$ .

**3.** Ventilator (1) as claimed in any of the preceding claims, wherein during use the pressure sensor (8) is arranged endotracheally.

**4.** Ventilator (1) as claimed in any of the preceding claims, wherein at least steps a) to c) are carried out in different pressure intervals (11).

**5.** Ventilator (1) as claimed in any of the preceding claims, wherein the pressure interval (11) encompasses at most 10 mbar.

**6.** Ventilator (1) as claimed in any of the preceding claims, wherein the fluid volume supplied or discharged within the pressure interval (11) is at most 10% of a maximum volume of the airway (5).

**7.** Ventilator (1) as claimed in any of the preceding claims, wherein at least five inspiration processes (13) and expiration processes (14) are carried out in step b).

8. Ventilator (1) as claimed in any of the preceding claims, wherein values for the pressure (9) and the fluid flow (4, 6) are sensed at the same time points in each case in step c) and the time points have time intervals of at most 0.1 seconds.

9. Ventilator (1) as claimed in any of the preceding claims, wherein the ventilator (1) is suitably designed for sole ventilation of the patient; wherein normoventilation of the patient is performable via the control device (10) at least before step a) or after step c).

10. Ventilator (1) as claimed in any of the preceding claims, wherein the gas discharge device (3) comprises a suction device, so that in step b) the second fluid flow (6) is at least partially generated by suction in at least an expiration process (14).

11. Ventilator (1) as claimed in any of the preceding claims, wherein the fluid flows (4, 6) are of equal size.

## Revendications

1. Ventilateur (1), comprenant au moins un dispositif d'alimentation en gaz (2) et un dispositif d'évacuation de gaz (3), destiné à délivrer un premier courant de fluide (4) aux voies respiratoires (5) d'un patient et à évacuer un second courant de fluide (6) à partir des voies respiratoires (5) de retour dans le ventilateur (1) ou vers une zone environnante (7), un détecteur de pression (8) pour détecter une pression $P_{trach}$ (9) dans les voies respiratoires (5) et un dispositif de commande (10) destiné à faire fonctionner le ventilateur (1) ; le dispositif de commande (10) étant configuré pour mettre en œuvre un procédé comprenant au moins les étapes suivantes :

   a) déterminer un intervalle de pression (11) dans lequel le patient doit respirer pendant un intervalle de temps déterminé (12) ;
   b) effectuer de manière alternée et répétée un processus d'inspiration (13) avec le premier courant de fluide (4) $Q_1$ au moyen du dispositif d'alimentation en gaz (2) et un processus d'expiration (14) avec le second courant de fluide (6) $Q_2$ au moyen du dispositif d'évacuation de gaz (3) à l'intérieur de l'intervalle de pression (11),
   c) détecter la pression (9) qui varie au cours de l'étape b) et les courants de fluide (4, 6) ;
   d) effectuer une transformation de Fourier sur les valeurs détectées de la pression (9) et établir un premier spectre de fréquence (15) pour la pression (9), et effectuer une transformation de Fourier sur les valeurs détectées des courants de fluide (4, 6) et établir un second spectre de fréquence (16) pour les courants de fluide (4, 6) ;
   e) calculer une impédance $Z_{aw}$ de la voie respiratoire (5) en divisant le premier spectre de fréquence (15) par le second spectre de fréquence (16), l'impédance comportant une composante réelle $Real(Z_{aw})$ (17) et une composante imaginaire $Im(Z_{aw})$ (18) ;
   f) simuler au moins la composante réelle (17) au moyen d'un premier modèle mathématique et déterminer une pression alvéolaire $P_{alv}$ (9) ou un profil d'une pression alvéolaire $P_{alv}$ (9) ;
   le premier modèle comprenant l'équation

$$Real(Z_{aw}) = R_{aw} + G/\omega^\alpha$$ , où $R_{aw}$ :

résistance liée aux voies respiratoires ;

$G/\omega^\alpha$ : résistance tissulaire ; où G est une constante, $\omega$ est la fréquence angulaire et $\alpha$ est une constante ; la composante réelle (17) décrivant la résistance, c'est-à-dire les résistances à surmonter pendant l'inspiration ou l'expiration ; où la pression alvéolaire $P_{alv}$ est déterminée à partir de l'équation $P_{alv} = P_{trach} - Q_1 \times R_{aw}$ ; avec $Q_i$ : le courant de fluide (4, 6) respectivement présent ;
   **caractérisé en ce que**, lors de l'étape f), la pression alvéolaire $P_{alv}$ (9) ou le profil de la pression alvéolaire $P_{alv}$ (9) est déterminé uniquement sur la base de la composante réelle (17) ; le courant de fluide (4, 6) pouvant être réglé à une valeur constante au moins pendant un processus d'inspiration (13) et un processus d'expiration (14) ; dans lequel, au cours de l'étape b), le premier courant de fluide (4) $Q_1$ et le second courant de fluide (6) $Q_2$ sont respectivement constants.

2. Dispositif de ventilation (1) selon la revendication 1 précédente, dans lequel, lors de l'étape f), la composante imaginaire (18) peut également être simulée par un second modèle mathématique, le second modèle comprenant l'équation

$$Z_{aw} = R_{aw} + k \times j \times \omega \times I_{aw} + \frac{G - j \times H}{\omega^\alpha}$$
: dans laquelle

$$Im(Z_{aw}) = j \times (k \times \omega \times I_{aw} + \frac{-H}{\omega^\alpha} ;$$

avec

   k : une constante ;
   $I_{aw}$ : inertie des voies respiratoires (5) ;

$\frac{-H}{\omega^\alpha}$ : Élasticité des voies respiratoires (5) où H est une constante ;

dans laquelle la partie imaginaire (18) décrit la réactance des voies respiratoires $X_a$, une compliance des voies respiratoires (5) étant décrite par $C = \frac{1}{\omega \times X_a}$.

3. Dispositif de ventilation (1) selon l'une quelconque des revendications précédentes, dans lequel le détecteur de pression (8) est disposé de manière endotrachéale lors de l'utilisation.

4. Dispositif de ventilation (1) selon l'une quelconque des revendications précédentes, dans lequel au moins les étapes a) à c) sont exécutées à des intervalles de pression différents (11).

5. Dispositif de ventilation (1) selon l'une quelconque des revendications précédentes, dans lequel l'intervalle de pression (11) est d'au plus 10 mbar.

6. Dispositif de ventilation (1) selon l'une quelconque des revendications précédentes, dans lequel le volume de fluide amené ou évacué à l'intérieur de l'intervalle de pression (11) est au maximum de 10 % d'un volume maximal de la voie respiratoire (5).

7. Dispositif de ventilation (1) selon l'une quelconque des revendications précédentes, dans lequel lors de l'étape b), au moins cinq processus d'inspiration (13) et processus d'expiration (14) sont effectués.

8. Dispositif de ventilation (1) selon l'une quelconque des revendications précédentes, **caractérisé en ce que**, lors de l'étape c), des valeurs pour la pression (9) et le courant de fluide (4, 6) sont détectées à des moments respectivement identiques, et les moments présentent des intervalles de temps d'au plus 0,1 seconde.

9. Dispositif de ventilation (1) selon l'une quelconque des revendications précédentes, dans lequel le dispositif de ventilation (1) est conçu pour être adapté à une ventilation exclusive du patient, une ventilation normale du patient pouvant être effectuée par l'intermédiaire du dispositif de commande (10) au moins temporairement avant l'étape a) ou après l'étape c).

10. Ventilateur (1) selon l'une quelconque des revendications précédentes, dans lequel le dispositif d'évacuation de gaz (3) comporte un dispositif d'aspiration, de sorte que, lors de l'étape b), le second courant de fluide (6) est généré au moins partiellement par aspiration lors d'au moins un processus d'expiration (14).

11. Dispositif de ventilation (1) selon l'une quelconque des revendications précédentes, **caractérisé en ce que** les courants de fluide (4, 6) sont de même amplitude.

**Fig. 1**

**Fig. 2**

**Fig. 3**

**Fig. 4**

**Fig. 5**

**Fig. 6**

## EP 4 181 991 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102016103678
- DE 102016109528

- DE 102007013385

**In der Beschreibung aufgeführte Nicht-Patentliteratur**

- **VON DAVID W. KACZKA et al.** *Oscillation Mechanics of the Respiratory System: Applications to Lung Disease*, 01 January 2011

- **BEI**. FLow-controlled EXpiration. *FLEX; s. Minerva Anestesiologica*, 2014, vol. 80 (1), 19-28

18